# EUROPEAN PATENT APPLICATION

(11) **EP 2 583 642 A1**
(43) Date of publication of application: **24.04.2013**
(21) Application number: 11008455.5
(22) Date of filing: 21.10.2011
(51) Int. Cl.: A61F 5/445, A61B 17/064, A61F 2/00, A61M 39/02, A61B 17/115

(54) **Implantable stoma ring**

(71) Applicant: Universitätsspital Basel, 4031 Basel (CH)
(72) Inventor: Kirchhoff, Philipp, 4055 Basel (CH)
(74) Representative: Kasche, André

(57) **Abstract**

The present invention relates to a ring (1) for implantation in patients receiving stoma having (i) an inner ring (2) suitable for fitting an abdominal wall on its outer circumference having an opening (3) suitable for an abdominal organ to pass through the ring to the exterior side (5) and (ii) a lip seal (4) extending over the interior side (5) of the inner ring (2) to seal the stoma opening for use in abdominal surgery. In addition, the present invention is directed to a method of treatment, comprising the surgical introduction of a ring (1) of the invention into the abdominal wall of a mammal in need of abdominal, preferably stoma surgery.

## Description

The present invention relates to a ring for implantation in patients receiving stoma having (i) an inner ring suitable for fitting an abdominal wall on its outer circumference having an opening suitable for an abdominal organ to pass through the ring to the exterior side and (ii) a lip seal extending over the interior side of the inner ring to seal the stoma opening for use in abdominal surgery. In addition, the present invention is directed to a method of treatment, comprising the surgical introduction of a ring of the invention into the abdominal wall of a mammal in need of abdominal, preferably stoma surgery.

### Background of the invention

A hernia is the protrusion of an organ or the fascia of an organ through the wall of a cavity that normally contains it. By far the most common herniae develop in the abdomen, when a weakness in the abdominal wall evolves into a localized hole, or "defect", through which adipose tissue or abdominal organs covered with peritoneum, may protrude.

An enterostoma is a surgically produced opening for intestinal parts through the abdominal wall. However, an enterostoma is often accompanied by herniation, i.e. the debility of the abdominal wall over time after stoma formation. The prevention of hernia development in enterostoma has only recently received scientific attention. The most encouraging results have included the use of a mesh inserted during the primary stoma operation. These studies demonstrated a prophylactic effect for the incorporation of a sublay light weight mesh installed during primary stoma surgery. The results are promising for the prevention of parastomal hernias, although the risk of organ adhesion to the mesh remains, long term complications have not yet been investigated and mesh introduction remains to be a demanding surgical procedure.

Each year about 100,000 new cases of colorectal cancer occur in Germany alone.

For 5 to 10 percent of these cases the surgical treatment requires the formation of a permanent stoma. As mentioned above, the trend of parastomal hernia repair is the insertion of a lightweight mesh. However, these mesh implants cause strong fibrosis, adhesion of the intestine or shrinkage of the implant, and in rare cases even perforation of the intestinal wall. Synthetic mesh is also associated with immunity-related complications. In phase I clinical studies biological mesh composed of non-cellular, cross-linked collagen shows promise for preventing parastomal hernias and avoiding immunity-related problems.

The objective technical problem underlying the present invention is the prevention of parastomal hernia in abdominal surgery, in particular the provision of medical devices/implants which can reduce the risk of or even permanently prevent parastomal hernia.

This problem is solved according to the claims by the provision of a ring for implanttation in patients receiving stoma consisting of (i) an inner ring suitable for fitting an abdominal wall on its outer circumference having an opening suitable for an abdominal organ to pass through the ring to the exterior side; and a lip seal extending over the interior side of the inner ring to seal the stoma opening, preferably towards the peritoneum, for use in abdominal surgery.

The ring of the invention is designed for implantation in patients, in particular into mammalian such as human patients. It is shaped to avoid mechanical stress, in particular tension and injury to the biological tissues in contact with it, e.g. it has no sharp edges, and it is made of material suitable for surgical introduction into and maintenance in living tissue; preferably it is made of biocompatible, non-degradable and/or easily sterilized material. It has an inner ring suitable for fitting the abdominal wall on its outer circumference and for providing an aperture on the inner side through which an intestinal bowel can be guided.

The inner ring is round, oval or preferably circular, although it can also have a round(ed)-like shape to the extent that its inner shape does not impose mechanical stress on the abdominal organ tissue placed through the opening of the inner ring and to the extent that its exterior shape does not impose mechanical stress on the abdominal wall In contact with and fixed to the Inner ring. In essence, the Inner ring is a narrow tube guide for receiving an abdominal organ, preferably an intestine or ureter on the inside and abdominal tissue on the outside. The inner ring has an interior side directed to the abdominal inside, and an exterior side directed to the stoma aperture. On the interior side of the inner ring there is a lip seal, e.g. a flange, which - once positioned and fixated surgically - seals the ring to the abdominal cavity to prevent leakage from the inside (interior) or contamination from the outside (exterior).

Of course, the dimensions of the inner ring, its opening, its width and thickness will vary with the sizes of the stoma and the intestine to be guided through the stoma. In a preferred embodiment for human use the ring of the invention is one consisting of (i) an inner ring having an opening of 2 to 6, preferably 2.5 to 5.5, more preferably 2 to 5, most preferably of about 2, 3, 4, 5 or 6 cm; a width of 3 to 20, preferably 5 to 15, more preferably 6 to 12, most preferably 7 to 10 mm; and a thickness of 1 to 15, preferably 1 to 12, more preferably 2 to 10, most preferably about 8 mm; and (ii) a ring-shaped lip seal extending 0.2 to 20 mm over the interior side of the inner ring.

In a preferred embodiment the rings according to the invention are used in stoma surgery including all kinds of abdominal stomas such as bowel and ureter (permanent, end/double and temporary) stomas.

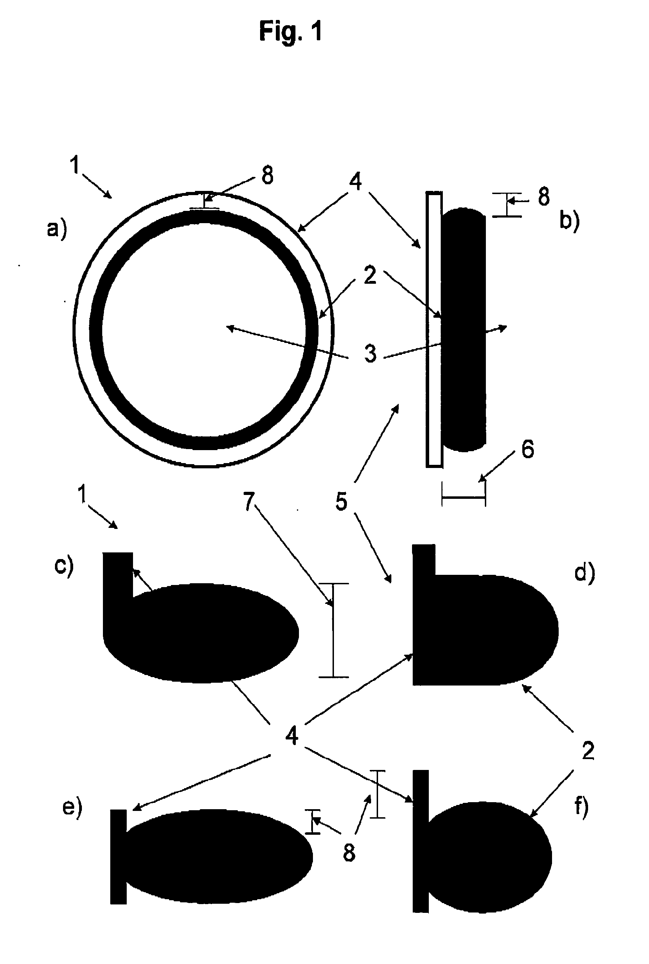

Preferably the inner ring has an outward curved cross-section, preferably an outward convex cross-section, more preferably has an oval cross section. An outward curved cross section of the inner ring has the advantage that the contact surface of the ring to the abdominal wall is increased and that the mechanical stress is reduced at the contact area of inner ring and lip seal. Preferred shapes of the inner ring are shown in Figs. 1 c to 1 e below.

It is also preferred that the ring of the invention is flexible but not dilatable in order that the ring can compensate movements of the adjacent abdominal wall but will not adapt its diameter due to external stress, e.g. temperature or muscle contraction.

The ring of the invention needs to be surgically or adhesively hold in place, i.e. fixated to the surrounding abdominal wall. It is therefore preferred that the ring material will allow suturing the ring to the adjacent abdominal wall. More preferably at least part of the ring, most preferably all of the ring is braided material to facilitate suturing.

In a further preferred embodiment at least part of the ring is made from biocompatible material, preferably from biocompatible polyethylene glycol terephthalate or polytetrafluorethylene, more preferably Dacron™ and Teflon™.

In a preferred embodiment it is also of advantage that at least part of the ring, preferably at least the part directly in contact with the abdominal wall and/or the abdominal organ is antiseptic, preferably coated with an antiseptic drug, more preferably triclosane.

In a further preferred embodiment, for temporarily or permanently fixating the ring of the invention at least part of the ring, preferably at least the part directly in contact with the abdominal wall and/or the abdominal organ is adhesive or coated with an adhesive. For example, parts of the ring can be temporarily adhesive to hold the ring in place for suturing.

In a further very preferred embodiment the ring of the invention is elastic and dilatable and/or constrictable to adapt the opening's diameter, preferably by loosening or tightening a thread in the inner ring and/or the lip seal. In most cases a thread in the inner ring will suffice to constrict the ring to the desired size. Once the desired opening size is set, the size can be fixed, e.g. by fastening the thread with a knot or by using other conventional techniques. This embodiment allows for closely adapting the opening of the ring to the actual size of the abdominal aperture. It also allows conveniently for correcting unintentional mistakes with regard to the aperture size. Fewer pre-sized rings or just one elastic ring can be used. In addition, the ring can be better adapted to the actual ring size.

The rings of the invention have a number of advantages over previous devices, e.g. light weight mesh, for preventing and/or treating parastomal hernia. The ring prevents the debility of the abdominal wall over time after stoma formation. Without the ring the abdominal sheaths of the muscle retract and the herniation proceeds. By fixating the layers of the abdominal wall to the inner ring the axial forces are evenly distributed and the weakening of the abdominal sheath and therefore forming of a hernia can be avoided. Also, the rings of the invention can be positioned and introduced in a rather simple and fast manner without extended subcutaneous tissue mobilisation, in particular when compared to the surgical mesh technique. They eliminate follow-up surgery, are cost-efficient in production, lower patient discomfort and reduce the time of hospitalisation.

Another aspect of the present invention is directed to a surgical stapler or suturing device comprising a ring according to the invention and means for clamping and/or suturing the ring to the walls of an abdominal aperture as well as optionally means for cutting a round aperture. Stapler or suturing devices for cutting, suturing and/or clamping living tissue are common in the art, e.g. are commercially available from Ethicon, (Curved Intraluminal Stapler (ILS), 33 mm diameter, with Adjustable Height Staples) and Covidien (EEA™ 21 mm Single-Use Stapler with 4.8 mm Staples). For producing surgical stapler and suturing devices according to the invention, common staplers and suturing devices can be routinely adapted to fit the rings of the present invention. The term "surgical stapler or suturing device" according to the invention also comprises a kit of parts comprising a ring according to the invention and means for clamping and/or suturing the ring to the walls of an abdominal aperture as well as optionally means for cutting a round aperture.

In a further aspect, the present invention is directed to a method of treatment, comprising the surgical introduction of a ring of the invention into the abdominal wall of a mammal in need of abdominal, preferably stoma surgery.

In a preferred embodiment this method comprises the following steps:
(i) incising the abdominal wall of said mammal to make an abdominal aperture, preferably a round abdominal aperture;
(ii) selecting a ring according to the present invention, so that the abdominal aperture fits the diameter of the outer surface of said inner ring;
(iii) fixating the ring in the abdominal aperture;
(iv) guiding an end of an abdominal organ, preferably of an intestine or ureter, through the opening of the inner ring;
(v) fixating the abdominal organ to form the external orifice.

It is preferred that the above method of the invention is performed on a mammal selected from the group consisting of cattle, equine, swine, ovine, canine, feline, camel and human.

In the following the present invention will be described and illustrated further with reference to specific examples and figures, none of which are considered as limiting the scope of the present invention as indicated by the appended claims.

### Figures

Fig. 1 depicts specific embodiments of the rings of the present invention.
Figs. 1a and 1 b are front and side views of a ring (1).
Figs. 1c to 1 e are side views of the ring (1) illustrating different inner ring shapes. Figure legend

| | | | |
|---|---|---|---|
| 1 | ring | 2 | inner ring |
| 3 | opening of inner ring | 4 | lip seal (flange) |
| 5 | interior side | 6 | width of (2) |
| 7 | thickness of (2) | 8 | extension of lip seal over (2) |

Figs.1a and 1 b depict a ring of the invention (1) comprising an inner ring (2) and a lip seal (flange) (4). The inner ring (2) has an opening (aperture) (3) which will receive and fixate the abdominal organ, preferably an intestine or ureter, for the enterostoma. The opening (3) for use in humans is preferably about 20, 30, 40 or 50 mm. The width of the inner ring is preferably 3 to 20 mm, enough to receive and fixate the abdominal wall on the outer circumference of the inner ring (2). Preferably, the opening (3) of the inner ring (2) can be adapted by constricting or expanding the inner ring (2). For example, a thread located in the inside or on the circumference of the inner ring (2) can be shortened and fastened to constrict the ring permanently to the desired exact size of the aperture in the abdominal wall. The ring-shaped lip seal (4) extends preferably about 0.2 to 20 mm (8) over the interior side of the inner ring (2) to seal the stoma opening against leaks from the inside and contamination from the outside.

The lip seal (4) and the inner ring (2) are preferably flexible to adapt to mechanical stress on the attached abdominal wall. The shape of the lip seal is functional, i.e. sealing and non-injuring. The shape of the inner ring (2) preferably has an outwardly curved cross-section, preferably an outward convex cross-section, more preferably has an oval cross section. Examples of preferred specific shapes of the inner ring are shown in Figs. 1c to 1e. The inner rings of Figs. 1c, 1e and 1f have in common that the outside circumference is outwardly curved and that the exterior side(s) of the inner rings are narrower than the middle section. This convex shape allows for increasing the contact area of the outer inner ring with the abdominal wall without changing the width of the inner ring and reduces tensional stress at the contact area of inner ring and lip seal, thus restricting the tensional stress of the abdominal wall to the middle of the inner ring. For easier suturing and/or clamping the ring (1) or at least the seal lip (4) and/or the inner ring (2) can be made from braided materials. Preferably the material is biocompatible and/or antiseptic, e.g. by nature or rendered by coating. For temporary or permanent fixation the part(s) directly in contact with the abdominal wall and/or abdominal organ are preferably adhesive or coated with an adhesive.

An exemplary procedure for introducing a ring (1) surgically into a patient is as follows: First an incision is made into the skin of the abdominal wall. Secondly a further round incision is made into the external rectus fascia. The muscle is preferably preserved by blunt separation and then the posterior rectus sheath is incised in order to produce a round abdominal aperture. Thirdly, a ring (1) is selected that fits the abdominal aperture. Then the ring is fixated to both abdominal sheaths by a circular suture with a non-absorbable thread. At last, the abdominal organ is pulled through the ring and fixated as usually to form the external orifice.

## Claims

1. A ring (1) for implantation in patients receiving stoma consisting of
(i) an inner ring (2) suitable for fitting an abdominal wall on its outer circumference having an opening (3) suitable for an abdominal organ to pass through the ring to the exterior side and
(II) a lip seal (4) extending over the interior side (5) of the inner ring (2) to seal the stoma opening
for use in abdominal surgery.

2. The ring (1) according to claim 1 consisting of
(i) an inner ring (2) having
(a) an opening (3) of 2 to 6, preferably 2.5 to 5.5, more preferably 2 to 5, most preferably of about 2, 3, 4, 5 or 6 cm;
(b) a width (6) of 3 to 20, preferably 5 to 15, more preferably 6 to 12, most preferably 7 to 10 mm;
(c) and a thickness (7) of 1 to 15, preferably 1 to 12, more preferably 2 to 10, most preferably about 8 mm;
(ii) a ring-shaped lip seal (4) extending 0.2 to 20 mm over the interior side (5) of the inner ring (2).

3. The ring (1) according to any of claims 1 and 2 for use in stoma surgery.

4. The ring (1) according to any of claims 1 to 3, wherein the inner ring (2) has an outward curved cross-section, preferably an outward convex cross-section, more preferably has an oval cross section.

5. The ring (1) according to any of claims 1 to 4, wherein the ring is flexible but not dilatable.

6. The ring (1) according to any of claims 1 to 5, wherein at least part of the ring is braided to allow suturing.

7. The ring (1) according to any of claims 1 to 6, wherein at least part of the ring is made from biocompatible material, preferably from biocompatible polyethylene glycol terephthalate or polytetrafluorethylen, more preferably Dacron™ and Teflon™.

8. The ring (1) according to any of claims 1 to 7, wherein at least part of the ring, preferably at least the part directly in contact with the abdominal wall and/or said abdominal organ is antiseptic, preferably coated with an antiseptic drug, more preferably triclosane.

9. The ring (1) according to any of claims 1 to 8, wherein at least part of the ring, preferably at least the part directly in contact with the abdominal wall and/or said abdominal organ is adhesive or coated with an adhesive.

10. The ring (1) according to any of claims 1 to 9, wherein the ring is elastic and dilatable and/or constrictable to adapt the opening's (3) diameter, preferably by loosening or tightening a thread in the inner ring (2) and/or lip seal (4).

11. Surgical stapler or suturing device comprising a ring (1) according to any of claims 1 to 10 and means for clamping and/or suturing the ring to the walls of an abdominal aperture as well as optionally means for cutting a round aperture.

12. A method of treatment, comprising the surgical introduction of a ring (1) according to any one of claims 1 to 10 into the abdominal wall of a mammal in need of abdominal, preferably stoma surgery.

13. A method according to claim 12, comprising the following steps:
(i) incising the abdominal wall of said mammal to make an abdominal aperture, preferably a round abdominal aperture;
(ii) selecting a ring (1) according to any one of claims 1 to 9, so that the abdominal aperture fits the diameter of the outer surface of said inner ring (2);
(iii) fixating the ring (1) in the abdominal aperture;
(iv) guiding an end of the abdominal organ through the opening (3) of the inner ring (2);
(v) fixating the abdominal organ to form the external orifice.

14. A method according to claim 12 or 13, wherein the mammal is selected from the group consisting of cattle, equine, swine, ovine, canine, feline, camel and human.
